# EUROPEAN PATENT APPLICATION

(11) **EP 4 461 722 A1**
(43) Date of publication of application: **13.11.2024**
(21) Application number: 22918377.7
(22) Date of filing: 14.12.2022
(51) Int. Cl.: C07C 45/00, C07C 49/04

(54) **METHOD FOR PREPARING METHYL ISOBUTYL KETONE**

(30) Priority: 04.01.2022 CN 202210005993
(71) Applicant: Sennics Co., Ltd., China (Shanghai) Pilot Free Trade Zone 200120 (CN)
(72) Inventor: MIAO, Zhengan, Shanghai 200120 (CN); LI, Shiwu, Shanghai 200120 (CN); XIANG, Yingjie, Shanghai 200120 (CN)
(74) Representative: V.O.
(86) International application number: PCT/CN2022/139038
(87) International publication number: WO 2023/130930

(57) **Abstract**

The present invention provides a method for preparing methyl isobutyl ketone. The method comprises: reacting mesityl oxide and methyl isobutyl alcohol under the action of a copper-based catalyst to produce methyl isobutyl ketone. In the present invention, mesityl oxide and methyl isobutyl alcohol are employed to carry out hydrogen in-situ transfer under the action of a copper-based catalyst, and methyl isobutyl ketone is prepared in one step. The entire reaction process does not require an external hydrogen system. The reaction is carried out under normal pressure and the process flow is short, has the advantages of high atom utilization, high safety and the like, and is suitable for popularization and industrial application.

## Description

### TECHNICAL FIELD

The present invention relates to a method for preparing methyl isobutyl ketone, specifically, to a method for preparing methyl isobutyl ketone from mesityl oxide and methyl isobutyl alcohol in one step.

### BACKGROUND

Methyl isobutyl ketone (MIBK), also commonly known as 4-methyl-2-pentanone, is a colorless, stable and flammable liquid with a pleasant odor. Its explosion range in the air is 1.4%~7.5%, thus it is strictly forbidden to get close to fire sources, and must be stored at low temperatures with sealed containers, avoiding long-term contact with skin. MIBK is slightly soluble in water, but completely miscible with some organic solvents, making it an excellent solvent. In recent years, due to the rapid development of the automobile industry, its market demand as a key raw material for preparing rubber antidegradant 6PPD has continued to grow.

At present, most companies use acetone as raw material, use acidic macroporous resin to support precious metal palladium catalyst, and synthesize MIBK through one-step condensation and hydrogenation in a fixed-bed reactor, and then separate and purify it through multiple towers to finally obtain MIBK products. For example, CN103274913A provides a methyl isobutyl ketone production method and equipment, and the method including the following steps: (a) acetone and hydrogen as raw materials are fed into a fixed bed tube reactor for reaction with a catalyst B621; the reaction products are first separated by condensation to obtain hydrogen and then the solution obtained by condensation is sent to a subsequent separation system; (b) the product solution is sent to a light component tower for separation, and the bottom liquid of the tower is sent to an acetone tower for separation; (c) the pressure of the acetone tower is controlled to a slight negative pressure, and the heavy components obtained at the bottom of the tower are sent to a dehydration tower for water removal; (d) the light components obtained from the top of the dehydration tower are sent to a wastewater tower for treatment to obtain organic light components; the product obtained at the bottom of the dehydration tower is sent to a product tower for purification, and the product methyl isobutyl ketone is obtained at the top of the product tower. Compared with the traditional one-step method, the average conversion rate of acetone has been improved, and the process is simple and by-products are less. Meanwhile it also improved the maximum recycling rate and energy consumption. However, due to the selection of catalysts and various parameters in process engineering are not perfect yet, it still leads to a lot of waste of energy and materials, high by-products, high operating temperature and pressure, and equipment investment and safety also need to be further improved. CN105130778A uses a strongly acidic cation exchange resin catalyst treated with palladium acetate to optimize the separation process and purify the by-product diisobutyl ketone (DIBK), achieving an increase in organic matter yield and a decrease in energy consumption. However, due to the use of precious metal palladium catalysts, the overall cost of synthesis is relatively high, and the reaction pressure is high, which resulted in large equipment investment and high overall production costs.

### SUMMARY OF THE INVENTION

In order to solve the deficiencies in the prior art, the present invention provides a new method for preparing methyl isobutyl ketone comprising new raw materials and new catalytic system, thereby achieving cost advantage. The present invention uses mesityl oxide (MO) and methyl isobutyl alcohol (MIBC) to transfer hydrogen in situ with a copper-based catalyst to prepare methyl isobutyl ketone in one step. The entire reaction process does not require an external hydrogen system. The reaction is carried out under normal pressure and the process flow is short. The method of the present application has the advantages of high atom utilization and high safety, and is suitable for industrial application.

Specifically, the present invention provides a method for preparing methyl isobutyl ketone, which comprises making mesityl oxide and methyl isobutyl alcohol react under action of a copper-based catalyst to generate methyl isobutyl ketone.

In some embodiments of the present invention, the copper-based catalyst comprises copper oxide, and a mass of copper oxide accounts for more than or equal to 50% of a total mass of the copper-based catalyst.

In some embodiments of the present invention, the copper-based catalyst is a copper-zinc-aluminum catalyst.

In some embodiments of the present invention, the copper-zinc-aluminum catalyst comprises copper oxide, zinc oxide, and aluminum oxide.

In some embodiments of the present invention, a mass of copper oxide accounts for more than or equal to 50% of a total mass of the copper-zinc-aluminum catalyst.

In some embodiments of the present invention, a mass of copper oxide is 50%-70% of a total mass of the copper-zinc-aluminum catalyst, a mass of zinc oxide is 10%-30% of a total mass of the copper-zinc-aluminum catalyst, and a mass of the aluminum oxide is 5%-15% of the total mass of a copper-zinc-aluminum catalyst.

In some embodiments of the present invention, the mass of copper oxide is 54%-64% of a total mass of the copper-zinc-aluminum catalyst, the mass of zinc oxide is 14%-24% of a total mass of the copper-zinc-aluminum catalyst, and the mass of the aluminum oxide is 7.8%-8% of a total mass of the copper-zinc-aluminum catalyst.

In some embodiments of the present invention, in the reaction, a feed molar ratio of mesityl oxide to methyl isobutyl alcohol is (0.1-2):1, preferably (0.8-1.2):1.

In some embodiments of the present invention, methyl isobutyl ketone is added to the reaction system at the beginning of the reaction.

In some embodiments of the present invention, a temperature of the reaction is from 140 °C to 250 °C, preferably 180±20 °C.

In some embodiments of the present invention, a pressure of the reaction is atmospheric pressure.

In some embodiments of the present invention, a volume space velocity of the reaction is 0.05-0.2 h⁻¹, preferably 0.1±0.02 h⁻¹.

In some embodiments of the present invention, hydrogen is not introduced during the reaction.

In some embodiments of the present invention, the reaction is performed in a fixed bed reactor, such as a single tube reactor.

In some embodiments of the present invention, the reaction is performed under protection of inert gas.

In some embodiments of the present invention, the method comprises: heating a reactor filled with a copper-based catalyst to a reaction temperature, feeding uniformly mixed mesityl oxide and methyl isobutyl alcohol from the bottom of the reactor for reaction, and discharging materials from the upper part of the reactor.

In some embodiments of the present invention, the uniformly mixed mesityl oxide and methyl isobutyl alcohol are preheated to the reaction temperature, and then fed to the reactor for reaction.

In some embodiments of the present invention, the reactor is a single-tube reactor with a diameter of 20±2 mm and a height of 700±100 mm, and a bed filling height of the copper-based catalyst is 500±50 mm.

### DESCRIPTION OF THE DRAWINGS

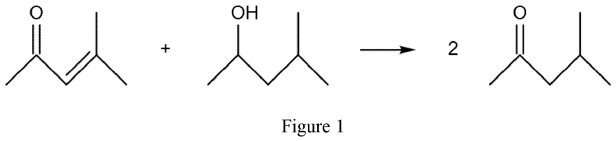

Figure 1 is a schematic diagram of the one-step preparation of methyl isobutyl ketone from mesityl oxide and methyl isobutyl alcohol.

### DETAILED DESCRIPTION OF THE INVENTION

In order to enable those skilled in the art to understand the characteristics and effects of the present invention, the terms and expressions mentioned in the description and claims are generally described and defined below. Unless otherwise specified, all technical and scientific terms used in the text have their usual meanings as understood by those skilled in the art regarding the present invention. In the event of conflict, the definitions in this specification shall prevail.

The theories or mechanisms described and disclosed herein, whether true or false, should not limit the scope of the invention in any way, that is, the invention may be implemented without being limited to any particular theory or mechanism.

In the present invention, "comprises", "includes", "contains" and similar terms encompass the meanings of "consisting essentially of" and "consisting of", for example, when it is disclosed herein that "A includes B and C", "A consists essentially of B and C" and "A consists of B and C" shall be deemed to have been disclosed herein.

In the present invention, all characteristics such as numerical values, amounts, contents, and concentrations defined in the form of numerical ranges or percentage ranges are for brevity and convenience only. Accordingly, descriptions of numerical ranges or percentage ranges shall be deemed to cover and specifically disclose all possible subranges and individual values within the ranges (including integers and fractions).

In the present invention, unless otherwise specified, percentage refers to mass percentage, and ratio refers to mass ratio.

In the present invention, when describing embodiments or examples, it should be understood that it is not intended to limit the present invention to these embodiments or examples. Conversely, all substitutes, modifications and equivalents of the methods and materials described in the present invention may be covered within the scope of the claims.

In the present invention, for the sake of conciseness, not all possible combinations of each technical feature in each embodiment or example are described. Thus, as long as there is no contradiction in the combination of these technical features, each technical feature in each embodiment or example may be arbitrarily combined, and all possible combinations should be considered to be within the scope of the specification.

In the present invention, the calculation formula for the conversion rate of methyl isobutyl alcohol (MIBC) is: MIBC conversion rate = (1 - MIBC residual amount/MIBC input amount) × 100%. The calculation formula for the conversion rate of mesityl oxide (MO) is: MO conversion rate = (1 - MO residual amount/MO input amount) × 100%. The calculation formula for the selectivity of methyl isobutyl ketone (MIBK) is: MIBK selectivity = MIBK production amount/ (MIBC conversion amount × n (MIBK)/n (MIBC) + MO conversion amount × n (MIBK)/n (MO))×100%, where n represents the amount of substance.

The present invention provides a new method for preparing methyl isobutyl ketone comprising new raw materials and new catalytic system. As shown in Figure 1, the present invention uses mesityl oxide (MO) and methyl isobutyl alcohol (MIBC) to transfer hydrogen in situ under action of a copper-based catalyst to prepare methyl isobutyl ketone in one step. The entire reaction process does not require an external hydrogen system. The reaction is carried out under normal pressure and the process flow is short. The method of the present application has the advantages of high atom utilization and high safety, and is suitable for industrial application.

The method for preparing methyl isobutyl ketone of the present invention comprises subjecting mesityl oxide and methyl isobutyl alcohol to an in-situ hydrogen transfer reaction under the action of a copper-based catalyst.

In the present invention, the copper-based catalyst comprises copper oxide, and the mass of copper oxide accounts for more than or equal to 50% of the total mass of the copper-based catalyst, such as >50%, 54%, or 64%. The copper-based catalyst is preferably a copper-zinc-aluminum catalyst. The main components of the copper-zinc-aluminum catalyst are copper oxide, aluminum oxide and zinc oxide, and the remaining components can be activated carbon. In some embodiments of the present invention, the copper-zinc-aluminum catalyst consists of copper oxide, aluminum oxide, zinc oxide and activated carbon. In the copper-zinc-aluminum catalyst, a copper oxide content can be 50%-70% of the total mass of the catalyst, such as 52%, 54%, 60%, 64%, 65%, and preferably 54%-64%; a zinc oxide content can be 10%-30% of the total mass of the catalyst, such as 12%, 14%, 20%, 24%, 25%, and preferably 14%-24%; and an aluminum oxide content can be 5%-15% of the total mass of the catalyst, such as 7%, 8%, 9%, 10%, and preferably 7.8%-8%. Maintaining the composition and proportions of the catalyst within the above ranges is conducive to in-situ transfer of hydrogen and shortens the process flow. Copper-based catalysts suitable for the present invention, such as copper-zinc-aluminum catalysts, are commercially available. In some embodiments of the present invention, the copper-zinc-aluminum used has a copper oxide content of 54%, a zinc oxide content of 24%, and an aluminum oxide oxide content of 8%, or has a copper oxide content of 64%, a zinc oxide content of 14%, and an aluminum oxide content of 7.8%. The catalyst used in the present invention may be in a cylindrical shape.

In the present invention, a feed molar ratio of mesityl oxide to methyl isobutyl alcohol can be from 0.1:1 to 2:1, for example, from 0.5:1 to 1.5:1, and preferably from 0.8:1 to 1.2:1, for example, around 1:1. Adopting a preferred feeding molar ratio is beneficial to improving the conversion rate of mesityl oxide, methyl isobutyl alcohol and the selectivity of methyl isobutyl ketone.

In the present invention, methyl isobutyl ketone can optionally be present in the initial reaction system. For example, mesityl oxide, methyl isobutyl alcohol and methyl isobutyl ketone can be mixed and put into the reaction, or only mesityl oxide and methyl isobutyl alcohol can be mixed and put into the reaction. When the initial reaction system contains methyl isobutyl ketone, the initial moles of methyl isobutyl ketone can be 0.5 to 2 times, for example 1 time, the total moles of mesityl oxide and methyl isobutyl alcohol.

In the present invention, the reaction pressure can be normal pressure, which is highly safe.

In the present invention, the reaction temperature can be from 140 °C to 250 °C, such as 140 °C, 160 °C, 180 °C, 200 °C, or 250 °C, and preferably around 180 °C. The reaction volume space velocity can be 0.05-0.2 h⁻¹, such as 0.06 h⁻¹, 0.08 h⁻¹, 0.1 h⁻¹, 0.12 h⁻¹, or 0.15 h⁻¹, and preferably around 0.1 h⁻¹. Preparing methyl isobutyl ketone under the above reaction conditions, especially the preferred reaction conditions, is beneficial to improving the conversion rate of mesityl oxide and methyl isobutyl alcohol and the selectivity of methyl isobutyl ketone.

There is no need to introduce hydrogen gas during the reaction process of the present invention. The reaction is carried out under the protection of an inert gas, such as nitrogen. For example, an inert gas can be used to replace the air in the reactor before the reaction.

The reaction of the present invention is preferably carried out in a fixed bed reactor, that is, the catalyst is preferably fixedly filled in the reactor, and the flowing raw materials react as they pass through the catalyst. The fixed bed reactor can be a tubular reactor, such as a single-tube reactor.

In the present invention, the reactor filled with the copper-based catalyst can be heated to the reaction temperature, uniformly mixed mesityl oxide and methyl isobutyl alcohol are fed from the bottom of the reactor for reaction, and materials are discharged from the upper part of the reactor. The uniformly mixed mesityl oxide and methyl isobutyl alcohol can be preheated to the reaction temperature before being fed to the reactor. A single-tube reactor with a diameter of 20±2 mm and a height of 700±100 mm can be used, and the bed filling height of the copper-based catalyst can be 500±50 mm.

In some embodiments of the present invention, the method for preparing methyl isobutyl ketone of the present invention comprises: heating a reactor filled with a copper-based catalyst to the reaction temperature, then inserting inert gas to purge the reactor, and adding uniformly mixed mesityl oxide, methyl isobutyl alcohol and optional methyl isobutyl ketone to a preheating system to preheat them to the reaction temperature, and then feeding them to the reactor for reaction. The rate at which raw materials enter the preheating system can be 0.125-0.5 mL/min, such as 0.25 mL/min.

The present invention has the following beneficial effects:
1. In the present invention, the entire reaction process does not require an external hydrogen and high-pressure circulation systems. The reaction is carried out under normal pressure and is highly safe;
2. In the present invention, a relatively low-price copper-based catalyst is used to replace the expensive palladium precious metal catalyst, thus the production cost is significantly reduced;
3. The present invention is a brand-new synthetic method of MIBK, which is different from the traditional process, and the product components are relatively simple, and subsequent product separation and purification are easier to achieve.

The present invention is described in the following examples. These examples are merely illustrative and are not intended to limit the scope of the present invention. The methods and reagents used in the examples, unless otherwise stated, are conventional methods and reagents in the art. In the examples, the copper-zinc-aluminum columnar catalyst whose ratio of copper oxide, zinc oxide, and aluminum oxide is 54:24:8 is commercially available with a type of Sichuan Chemical CB-5; the copper-zinc-aluminum columnar catalyst whose ratio of copper oxide, zinc oxide, and aluminum oxide is 64:14:7.8 is commercially available with a type of Sichuan Chemical AF104.

### Example 1

A certain amount of copper-zinc-aluminum cylindrical catalyst (copper oxide content is 54%, zinc oxide content is 24%, aluminum oxide content is 8%) is loaded into a single-tube reactor with a diameter of 20 mm and a height of 700 mm, and the catalyst bed filling height is 500 mm. The heating system is turned on to raise the temperature of the reaction device. After the temperature reaches 140 °C, dry nitrogen is introduced from the top to purge the reactor for 10 min. MO and MIBC are mixed at a molar ratio of 1:1, then MIBK with the moles equal to the sum of the moles of MIBC and MO is added, and the materials are mixed evenly and pumped into the preheating system at a speed of 0.25 mL/min using a constant flow pump. After the mixture is preheated to 140 °C, it is fed to the reactor from the bottom of the reactor and the materials are discharged from the upper part of the reactor with a reaction volume space velocity of 0.1 h⁻¹, and after condensation, samples are analyzed by GC. The MIBC conversion rate is 82.14%, the MO conversion rate is 80.1%, and the MIBK selectivity is 98.60%.

### Example 2

A certain amount of copper-zinc-aluminum cylindrical catalyst (copper oxide content is 54%, zinc oxide content is 24%, aluminum oxide content is 8%) is loaded into a single-tube reactor with a diameter of 20 mm and a height of 700 mm, and the catalyst bed filling height is 500 mm. The heating system is turned on to raise the temperature of the reaction device. After the temperature reaches 160 °C, dry nitrogen is introduced from the top to purge the reactor for 10 min. MO and MIBC are mixed at a molar ratio of 1:1, then MIBK with the moles equal to the sum of the moles of MIBC and MO is added, and the materials are mixed evenly and pumped into the preheating system at a speed of 0.25 mL/min using a constant flow pump. After the mixture is preheated to 160 °C, it is fed to the reactor from the bottom of the reactor and the materials are discharged from the upper part of the reactor with a reaction volume space velocity of 0.1 h⁻¹, and after condensation, samples are analyzed by GC. The MIBC conversion rate is 90.14%, the MO conversion rate is 89%, and the MIBK selectivity is 98.52%.

### Example 3

A certain amount of copper-zinc-aluminum cylindrical catalyst (copper oxide content is 54%, zinc oxide content is 24%, aluminum oxide content is 8%) is loaded into a single-tube reactor with a diameter of 20 mm and a height of 700 mm, and the catalyst bed filling height is 500 mm. The heating system is turned on to raise the temperature of the reaction device. After the temperature reaches 180 °C, dry nitrogen is introduced from the top to purge the reactor for 10 min. MO and MIBC are mixed at a molar ratio of 1:1, then MIBK with the moles equal to the sum of the moles of MIBC and MO is added, and the materials are mixed evenly and pumped into the preheating system at a speed of 0.25 mL/min using a constant flow pump. After the mixture is preheated to 180 °C, it is fed to the reactor from the bottom of the reactor and the materials are discharged from the upper part of the reactor with a reaction volume space velocity of 0.1 h⁻¹, and after condensation, samples are analyzed by GC. The MIBC conversion rate is 96.14%, the MO conversion rate is 94.23%, and the MIBK selectivity is 98.33%.

### Example 4

A certain amount of copper-zinc-aluminum cylindrical catalyst (copper oxide content is 54%, zinc oxide content is 24%, aluminum oxide content is 8%) is loaded into a single-tube reactor with a diameter of 20 mm and a height of 700 mm, and the catalyst bed filling height is 500 mm. The heating system is turned on to raise the temperature of the reaction device. After the temperature reaches 200 °C, dry nitrogen is introduced from the top to purge the reactor for 10 min. MO and MIBC are mixed at a molar ratio of 1:1, then MIBK with the moles equal to the sum of the moles of MIBC and MO is added, and the materials are mixed evenly and pumped into the preheating system at a speed of 0.25 mL/min using a constant flow pump. After the mixture is preheated to 200 °C, it is fed to the reactor from the bottom of the reactor and the materials are discharged from the upper part of the reactor with a reaction volume space velocity of 0.1 h⁻¹, and after condensation, samples are analyzed by GC. The MIBC conversion rate is 97.21%, the MO conversion rate is 95.72%, and the MIBK selectivity is 96.01%.

### Example 5

A certain amount of copper-zinc-aluminum cylindrical catalyst (copper oxide content is 54%, zinc oxide content is 24%, aluminum oxide content is 8%) is loaded into a single-tube reactor with a diameter of 20 mm and a height of 700 mm, and the catalyst bed filling height is 500 mm. The heating system is turned on to raise the temperature of the reaction device. After the temperature reaches 250 °C, dry nitrogen is introduced from the top to purge the reactor for 10 min. MO and MIBC are mixed at a molar ratio of 1:1, then MIBK with the moles equal to the sum of the moles of MIBC and MO is added, and the materials are mixed evenly and pumped into the preheating system at a speed of 0.25 mL/min using a constant flow pump. After the mixture is preheated to 250 °C, it is fed to the reactor from the bottom of the reactor and the materials are discharged from the upper part of the reactor with a reaction volume space velocity of 0.1 h⁻¹, and after condensation, samples are analyzed by GC. The MIBC conversion rate is 98.59%, the MO conversion rate is 97.31%, and the MIBK selectivity is 92.15%.

### Example 6

A certain amount of copper-zinc-aluminum cylindrical catalyst (copper oxide content is 54%, zinc oxide content is 24%, aluminum oxide content is 8%) is loaded into a single-tube reactor with a diameter of 20 mm and a height of 700 mm, and the catalyst bed filling height is 500 mm. The heating system is turned on to raise the temperature of the reaction device. After the temperature reaches 180 °C, dry nitrogen is introduced from the top to purge the reactor for 10 min. MO and MIBC are mixed at a molar ratio of 0.1:1, then MIBK with the moles equal to the sum of the moles of MIBC and MO is added, and the materials are mixed evenly and pumped into the preheating system at a speed of 0.25 mL/min using a constant flow pump. After the mixture is preheated to 180 °C, it is fed to the reactor from the bottom of the reactor and the materials are discharged from the upper part of the reactor with a reaction volume space velocity of 0.1 h⁻¹, and after condensation, samples are analyzed by GC. The MIBC conversion rate is 54.32%, the MO conversion rate is 99.33%, and the MIBK selectivity is 98.15%.

### Example 7

A certain amount of copper-zinc-aluminum cylindrical catalyst (copper oxide content is 54%, zinc oxide content is 24%, aluminum oxide content is 8%) is loaded into a single-tube reactor with a diameter of 20 mm and a height of 700 mm, and the catalyst bed filling height is 500 mm. The heating system is turned on to raise the temperature of the reaction device. After the temperature reaches 180 °C, dry nitrogen is introduced from the top to purge the reactor for 10 min. MO and MIBC are mixed at a molar ratio of 2:1, then MIBK with the moles equal to the sum of the moles of MIBC and MO is added, and the materials are mixed evenly and pumped into the preheating system at a speed of 0.25 mL/min using a constant flow pump. After the mixture is preheated to 180 °C, it is fed to the reactor from the bottom of the reactor and the materials are discharged from the upper part of the reactor with a reaction volume space velocity of 0.1 h⁻¹, and after condensation, samples are analyzed by GC. The MIBC conversion rate is 99.32%, the MO conversion rate is 45.22%, and the MIBK selectivity is 97.26%.

### Example 8

A certain amount of copper-zinc-aluminum cylindrical catalyst (copper oxide content is 54%, zinc oxide content is 24%, aluminum oxide content is 8%) is loaded into a single-tube reactor with a diameter of 20 mm and a height of 700 mm, and the catalyst bed filling height is 500 mm. The heating system is turned on to raise the temperature of the reaction device. After the temperature reaches 180 °C, dry nitrogen is introduced from the top to purge the reactor for 10 min. MO and MIBC are mixed at a molar ratio of 1:1, then MIBK with the moles equal to the sum of the moles of MIBC and MO is added, and the materials are mixed evenly and pumped into the preheating system at a speed of 0.125 mL/min using a constant flow pump. After the mixture is preheated to 180 °C, it is fed to the reactor from the bottom of the reactor and the materials are discharged from the upper part of the reactor with a reaction volume space velocity of 0.05 h⁻¹, and after condensation, samples are analyzed by GC. The MIBC conversion rate is 98.89%, the MO conversion rate is 97.77%, and the MIBK selectivity is 96.91%.

### Example 9

A certain amount of copper-zinc-aluminum cylindrical catalyst (copper oxide content is 54%, zinc oxide content is 24%, aluminum oxide content is 8%) is loaded into a single-tube reactor with a diameter of 20 mm and a height of 700 mm, and the catalyst bed filling height is 500 mm. The heating system is turned on to raise the temperature of the reaction device. After the temperature reaches 180 °C, dry nitrogen is introduced from the top to purge the reactor for 10 min. MO and MIBC are mixed at a molar ratio of 1:1, then MIBK with the moles equal to the sum of the moles of MIBC and MO is added, and the materials are mixed evenly and pumped into the preheating system at a speed of 0.5 mL/min using a constant flow pump. After the mixture is preheated to 180 °C, it is fed to the reactor from the bottom of the reactor and the materials are discharged from the upper part of the reactor with a reaction volume space velocity of 0.2 h⁻¹, and after condensation, samples are analyzed by GC. The MIBC conversion rate is 87.32%, the MO conversion rate is 83.15%, and the MIBK selectivity is 98.02%.

### Example 10

A certain amount of copper-zinc-aluminum cylindrical catalyst (copper oxide content is 64%, zinc oxide content is 14%, aluminum oxide content is 7.8%) is loaded into a single-tube reactor with a diameter of 20 mm and a height of 700 mm, and the catalyst bed filling height is 500 mm. The heating system is turned on to raise the temperature of the reaction device. After the temperature reaches 180 °C, dry nitrogen is introduced from the top to purge the reactor for 10 min. MO and MIBC are mixed at a molar ratio of 1:1, then MIBK with the moles equal to the sum of the moles of MIBC and MO is added, and the materials are mixed evenly and pumped into the preheating system at a speed of 0.25 mL/min using a constant flow pump. After the mixture is preheated to 180 °C, it is fed to the reactor from the bottom of the reactor and the materials are discharged from the upper part of the reactor with a reaction volume space velocity of 0.1 h⁻¹, and after condensation, samples are analyzed by GC. The MIBC conversion rate is 97.5%, the MO conversion rate is 96.83%, and the MIBK selectivity is 98.22%.

### Example 11

A certain amount of copper-zinc-aluminum cylindrical catalyst (copper oxide content is 64%, zinc oxide content is 14%, aluminum oxide content is 7.8%) is loaded into a single-tube reactor with a diameter of 20 mm and a height of 700 mm, and the catalyst bed filling height is 500 mm. The heating system is turned on to raise the temperature of the reaction device. After the temperature reaches 180 °C, dry nitrogen is introduced from the top to purge the reactor for 10 min. MO and MIBC are mixed at a molar ratio of 1:1, and the mixture is pumped into the preheating system at a speed of 0.25 mL/min using a constant flow pump. After the mixture is preheated to 180 °C, it is fed to the reactor from the bottom of the reactor and the materials are discharged from the upper part of the reactor with a reaction volume space velocity of 0.1 h⁻¹, and after condensation, samples are analyzed by GC. The MIBC conversion rate is 98.72%, the MO conversion rate is 97.93%, and the MIBK selectivity is 98.15%.

The examples above show that the present invention uses mesityl oxide and methyl isobutyl alcohol to transfer hydrogen in situ under the action of a copper-based catalyst to prepare methyl isobutyl ketone in one step. The entire reaction process does not require an external hydrogen system. The reaction is carried out under normal pressure and the process flow is short. It achieves good MIBC conversion rate, MO conversion rate and selectivity and has the advantages of high atom utilization and high safety, and is suitable for industrial application.

## Claims

1. A method for preparing methyl isobutyl ketone, comprising: making mesityl oxide and methyl isobutyl alcohol react under action of copper catalyst to generate methyl isobutyl ketone.

2. The method according to claim 1, wherein the copper-based catalyst comprises copper oxide, and a mass of copper oxide accounts for more than or equal to 50% of a total mass of the copper-based catalyst.

3. The method according to claim 1, wherein the copper-based catalyst is a copper-zinc-aluminum catalyst, and preferably, the copper-zinc-aluminum catalyst comprises copper oxide, zinc oxide and aluminum oxide.

4. The method according to claim 3, wherein in the copper-zinc-aluminum catalyst, a mass of copper oxide accounts for more than or equal to 50% of a total mass of the copper-zinc-aluminum catalyst;
preferably, in the copper-zinc-aluminum catalyst, a mass of copper oxide is 50%-70% of a total mass of the copper-zinc-aluminum catalyst, a mass of zinc oxide is 10%-30% of a total mass of the copper-zinc-aluminum catalyst, and a mass of the aluminum oxide is 5%-15% of the total mass of a copper-zinc-aluminum catalyst;
more preferably, in the copper-zinc-aluminum catalyst, a mass of copper oxide is 54%-64% of a total mass of the copper-zinc-aluminum catalyst, a mass of zinc oxide is 14%-24% of a total mass of the copper-zinc-aluminum catalyst, and a mass of the aluminum oxide is 7.8%-8% of a total mass of the copper-zinc-aluminum catalyst.

5. The method according to claim 1, wherein in the reaction, a feed molar ratio of mesityl oxide to methyl isobutyl alcohol is (0.1-2): 1, preferably (0.8-1.2): 1.

6. The method according to claim 1, wherein methyl isobutyl ketone is added to the reaction system at the beginning of reaction.

7. The method according to claim 1, wherein the reaction has one or more of the following characteristics:
a temperature of the reaction is 140°C-250 °C, preferably 180±20 °C;
a pressure of the reaction is normal pressure;
a volume space velocity of the reaction is 0.05-0.2 h⁻¹, preferably 0. 1±0.02 h⁻¹;
hydrogen is not introduced during the reaction;
the reaction is carried out in a fixed bed reactor, such as a single-tube reactor;
the reaction is carried out under protection of inert gas.

8. The method according to claim 1, wherein the method comprising: heating a reactor filled with a copper-based catalyst to a reaction temperature, feeding uniformly mixed mesityl oxide and methyl isobutyl alcohol from the bottom of the reactor for reaction and discharging materials from the upper part of the reactor.

9. The method according to claim 7, wherein the uniformly mixed mesityl oxide and methyl isobutyl alcohol are preheated to the reaction temperature before fed to the reactor for reaction.

10. The method according to claim 7, wherein the reactor is a single-tube reactor with a diameter of 20±2 mm and a height of 700±100 mm, and a bed filling height of the copper-based catalyst is 500±50 mm.
